# NEUE EUROPÄISCHE PATENTSCHRIFT

(11) **EP 0 705 846 B2**
(45) Veröffentlichungstag und Bekanntmachung des Hinweises auf die Entscheidung über den Einspruch: **29.10.2008**
(45) Hinweis auf die Patenterteilung: 26.05.2004
(21) Anmeldenummer: 95115442.6
(22) Anmeldetag: 29.09.1995
(51) Int. Cl.: C07K 14/755, A61K 38/37

(54) **Hochmolekulare und niedermolekulare Fraktionen des von Willebrand-Faktors**
High molecular weight and low molecular weight fractions of von Willebrand factor
Les fractions du facteur von Willebrand à haut poids moléculaires et à bas poids moléculaires

(30) Priorität: 04.10.1994 DE 4435392
(43) Veröffentlichungstag der Anmeldung: 10.04.1996
(73) Patentinhaber: Baxter Aktiengesellschaft, 1221 Wien (AT)
(72) Erfinder: Fischer, Bernhard, Dr., 1160 Wien (AT); Mitterer, Artur, Dr., 2304 Orth/Donau (AT); Dorner, Friedrich, Prof. Dr., A-1230 Wien (AT)
(74) Vertreter: Polz, Leo

(56) Entgegenhaltungen:
- JOURNAL OF CELL BIOLOGY, Bd. 101, Nr. 1, Juli 1985 Seiten 112-120, D.D. WAGNER ET AL. 'Inhibition of Disulfide Bonding of von Willebrand Protein by Monensin Results in Small, Functionally Defective Multimers'
- JOURNAL OF BIOLOGICAL CHEMISTRY. (MICROFILMS), Bd. 258, Nr. 20, 25.Oktober 1983 BALTIMORE, MD US, Seiten 12327-12333, S.E. SENOGLES UND G.L. NELSESTUEN 'von Willebrand Factor'
- FISCHER, BERNHARD ET AL: 'Structural analysis of recombinant von Willebrand factor: identification of hetero- and homo-dimers' FEBS LETT Bd. 351, Nr. 3, 12 September 1994, Seiten 345 - 348, XP002140917
- P. BAILLOD ET AL.: 'Multimeric Analysis of von Willebrand Factor by Vertical Sodium Dodecyl Sulphate Agarose Gel Electrophoresis, Vacuum Blotting Technology and Sensitive Visualization by Alkaline Phosphatase Anti-Alkaline Phosphatase Complex' THROMBOSIS RESEARCH Bd. 66, Nr. 6, 15 Juni 1992, Seiten 745 - 755, XP009023570
- BURNOUF-RADOSEVICH; BURNOUF: 'Chromatographic preparation of a therapeutic highly purified von Willebrand Factor concentrate from human cryoprecipitate' VOX SANG Bd. 62, 1992, Seiten 1 - 11
- CHOPEK M. ET AL: 'Human von Willebrand Factor: a multivalent protein composed of identical subunits' BIOCHEMISTRY Bd. 25, 1986, Seiten 3146 - 3155

## Beschreibung

Die Erfindung betrifft ein Verfahren zur Auftrennung von von Willebrand-Faktor in eine hochmolekulare und niedermolekulare Fraktion.

Weiterhin bezieht sich die Erfindung auf Zusammensetzungen zur medizinischen Verwendung, enthaltend eine niedermolekulare Fraktion von von Willebrand-Faktor (vWF) Molekülen, eine hochmolekulare Fraktion von von Willebrand-Faktor Molekülen, sowie eine Mischung aus vWF-Molekülen der niedermolekularen und hochmolekularen Fraktion, gemäß den Ansprüchen.

Dem von Willebrand-Faktor kommen in der normal ablaufenden Blutgerinnung direkte und indirekte Aufgaben zu. Er bindet in einem Komplex an den Faktor VIII. Dieser Komplex dient zur Stabilisierung des Faktors VIII. Dieser stabilisierte Faktor VIII hat dann wesentliche Cofaktorfunktion bei der Aktivierung von Faktor X. Der von Willebrand-Faktor greift aber auch direkt in die Blutgerinnung ein, indem er die Plättchenaggregation an verletzten Gefässen vermittelt.

Im Plasma zirkuliert der vWF in einer Konzentration von 5-10 mg/l in Form eines nicht-kovalenten Komplexes mit dem Faktor VIII. Der vWF ist ein Glycoprotein, welches in verschiedenen Zellen des menschlichen Körpers gebildet und später in die Zirkulation freigesetzt wird. Dabei wird in den Zellen ausgehend von einer Polypeptidkette mit einem Molekulargewicht von ca. 220000 (vWF-Monomer) durch Ausbildung von mehreren Schwefelbrücken ein vWF-Dimer (primäres vWF-Dimer) mit einem Molekulargewicht von ca. 550000 gebildet. Aus den vWF-Dimeren werden dann durch Verknüpfung weitere Polymere des vWF mit immer höheren Molekulargewichten bis zu ca. 20 Millionen hergestellt.

Es gibt mehrere Krankheitsbilder, die auf Unter- oder Überproduktion des von Willebrand-Faktors zurückzuführen sind. So führt zum Beispiel eine Überproduktion von vWF zur vermehrten Neigung von Thrombosen, wohingegen eine Unterversorgung mit vWF eine vermehrte Blutungsneigung oder verlängerte Blutungszeit zur Folge hat.

Das von Willebrand-Syndrom kann sich in mehreren Erscheinungsformen manifestieren. Alle Formen zeichnen sich durch eine verlängerte Blutungszeit aus, die entweder durch absolutes Fehlen eines funktionellen vWF oder durch ein abnormes Spektrum in der Multimerenzusammensetzung des vWF begründet ist. Dabei wurden sowohl Formen der von Willebrand Disease diagnostiziert, bei denen die Multimerenbildung reduziert ist, als auch Formen, bei denen die niedermolekularen vWF-Moleküle kaum vorhanden sind. Andere Formen wiederum zeigen zwar hoch- und niedermolekulare vWF-Moleküle, ihre Konzentration beziehungsweise ihr Verhältnis zueinander ist aber gegenüber einer gesunden Person drastisch verringert beziehungsweise verändert.

Der Mangel an vWF kann auch eine Hämophilie A hervorrufen, da der vWF, wie oben erwähnt, ein wesentlicher Bestandteil des funktionellen Faktors VIII ist. In diesen Fällen ist die Halbwertszeit des Faktors VIII derart verringert, dass er seine speziellen Funktionen in der Blutgerinnungskaskade nicht wahrnehmen kann.

Sowohl alle Erscheinungsformen des von Willebrand-Syndroms als auch die auf den Mangel an vWF zurückzuführende Form der Hämophilie A wurden bisher durch Ersatz des fehlenden vWF durch intravenöse Infusionen mit Konzentraten aus Blutplasma, die entweder vWF-Faktor VIII-Komplex oder angereicherten vWF enthalten, behandelt. In beiden Krankheitsfällen ist zwar einerseits die Gabe von Faktor VIII nicht notwendig, die präparative Abtrennung des vWF von Faktor VIII aber technisch sehr schwierig bis unmöglich.

Um die genaue Funktion der hochmolekularen vWF-Moleküle einerseits und der niedermolekularen vWF-Moleküle andererseits zu eruieren, suchte man Wege, diese Fraktionen in angereicherter Form zu gewinnen. Wagner D.D. et al (J. Cell Biol. 101:112, 1985) verhindern durch Zugabe von Monensin in vitro die Multimerenbildung und schliessen aus verschiedenen Versuchen, dass die niedermolekulare Form des vWF nicht funktionell ist. Dahingegen können Senogles S.E. et al (J. Biol. Chem. 258:12327, 1983) keine Funktionsunterschiede in der Ristocitin vermittelten Plättchenaggregation bei hochmolekularen und niedermolekularen Formen des vWF finden. Sie erhalten die niedermolekularen Formen durch Reduktion der Schwefelbrücken von hochmolekularen vWF-Molekülen. Aihara M. et al (Tohoku J. exp. Med. 153:169, 1985) beobachten eine geringere Bindungsfähigkeit der vWF-Moleküle von Patienten mit Willebrand-Syndrom der Type IIa an Collagen in einer Affinitätschromatographie. Diese Art des von Willebrand-Syndroms ist durch das Fehlen der hochmolekularen Moleküle des vWF charakterisiert.

In der Literatur gibt es zahlreiche Verfahren, die eine analytische Trennung von hochmolekularen und niedermolekularen Formen des von Willebrand-Faktors beschreiben, um Aussagen über das Mengenverhältnis der beiden Formen zuzulassen. Als Beispiel sei hier eine Publikation von Baillod et al in Thrombosis Res. 66:745, 1992 genannt. Desweiteren beschreibt Burnouf-Radosevich in Vox Sang 62:1-11, 1992 die Aufreinigung von vWF mittels lonenaustausch- und Affinitätschromatographie aus Cryopräzipitat. Die erhaltenen Fraktionen zeigten eine Anreicherung der größeren multimeren Formen im Vergleich zu normalem Plasma, was auf die lonenaustausch-Chromatographie zurückgeführt wurde. Die Aktivität des erhaltenen vWF Konzentrats in der Plättchenbindung lag bei 86% derjenigen von Plasma vWF. Ein präparatives Verfahren zur Trennung von niedermolekularen und hochmolekularen Formen wurde bisher jedoch nicht beschrieben.

Aufgabe der vorliegenden Erfindung ist es, ein präparatives Verfahren zur Auftrennung von von Willebrand- Faktor (vWF) in eine hochmolekulare und niedermolekulare Fraktion von von Willebrand-Faktor zu entwickeln.

Diese Aufgabe wird gelöst durch ein Verfahren zur Trennung von vWF, insbesondere von rekombinantem von Willebrand-Faktor, in hochmolekularen vWF und niedermolekularen vWF, das dadurch gekennzeichnet ist, dass man den vWF an einem Affinitätsträger bindet und dann bei unterschiedlichen Salzkonzentrationen eluiert.

Bevorzugte Ausführungsformen sind in den Unteransprüchen 2 bis 13 beschrieben.

Bei einer besonders bevorzugten Ausführungsform der vorliegenden Erfindung wird die Trennung in einem Ca²⁺-freien Puffersystem durchgeführt. Auf diese Weise lassen sich hochmolekulare vWF-Fraktionen oder hochmolekulare rvWF-Fraktionen, die eine besonders hohe physiologische Aktivität besitzen, mit guten Ausbeuten erhalten.

Weiterer Gegenstand der vorliegenden Erfindung ist eine Zusammensetzung zur medizinischen Verwendung enthaltend niedermolekulare Fraktion von vWF-Molekülen, die Dimere und Tetramere enthält, wobei die Dimere und Tetramere aus identischen vWF-Untereinheiten bestehen.

Bevorzugte Ausführungsformen sind in den Unteransprüchen 15 und 16 bis 19 beschrieben.

Die vorliegende Erfindung bezieht sich auch auf Zusammensetzung zur medizinischen Verwendung enthaltend eine hochmolekulare Fraktion von vWF-Molekülen, gemäß den Ansprüchen 16-18 die eine um mindestens 50%, vorzugsweise 60 % verbesserte Aktivität pro µg Protein in der Plättchenaggregation aufweist, verglichen mit der physiologischen Mischung von hochmolekularen und niedermolekularen vWF-Molekülen. Die Aktivität pro µg Protein kann erfindungsgemäss noch weiter gesteigert werden.

Bevorzugte Ausführungsformen sind in dem Unteransprüch 18 beschrieben.

Die Erfindung betrifft auch die Verwendung der medizinischen Zusammensetzung enthaltend die niedermolekulare Fraktion von vWF-Molekülen oder die hochmolekulare Fraktion von vWF-Molekülen gemäß den Ansprüchen zur Behandlung von Hämophilie A oder verschiedener Formen der von Willebrand Disease.

Es war im Hinblick auf den Stand der Technik nicht vorhersehbar, dass die nach einem präparativen Trennungsverfahren erhältlichen Fraktionen bzw. deren Mischung zur Behandlung der genannten Krankheiten geeignet sind.

Das erfindungsgemässe Verfahren eignet sich in gleicher Weise für plasmatischen wie auch rekombinanten vWF (rvWF). Ausgangsmaterial zur Trennung der hoch- und niedermolekularen Fraktionen ist entweder eine vWF angereicherte Plasmafraktion oder ein zellfreies Kulturmedium nach Fermentation tierischer Zellen, aus dem rekombinanter vWF isoliert und vorgereinigt wurde.

Der für die Trennung verwendete vWF kann mit Hilfe jedes bekannten Verfahrens vorgereinigt werden.

Gemäss dem erfindungsgemässen Verfahren zur Trennung von vWF und insbesondere rvWF in hochmolekulare und niedermolekulare Fraktionen wird der vWF an einem Affinitätsträger gebunden und dann bei unterschiedlichen Salzkonzentrationen eluiert.

Für die Erzielung besonders hoher Ausbeuten wird die Trennung in einem Ca²⁺-freien Puffersystem durchgeführt.

Die niedermolekularen vWF-Fraktionen lassen sich bei einer geringeren Salzkonzentration als die hochmolekularen vWF-Fraktiönen eluieren.

Für die Elution sind lösliche ein- und zweiwertige Salze verwendbar. Vorzugsweise wird NaCl verwendet. Calciumsalze sind für die Elution nicht geeignet.

In einer bevorzugten Ausführungsform wird der vWF bei einer Salzkonzentration von < 150 mM an den Affinitätsträger gebunden. Bei einer Salzkonzentration zwischen 150 und 250 mM, und insbesondere bei 160 mM, werden dann die niedermolekularen Aggregate des vWFs eluiert, und danach bei einer Salzkonzentration von > 250 mM und insbesondere bei ≥ 270 mM die hochmolekularen Aggregate eluiert.

Bevorzugt wird NaCl als Salz verwendet. Calciumsalze sind nicht geeignet.

Das erfindungsgemässe Verfahren wird vorzugsweise an einer Heparin-Affinitätschromatographie-Säule durchgeführt. Für die Affinitätschromatographie kann jeder Träger, an dem Heparin gebunden werden kann, verwendet werden. Als gut geeignet erwiesen sich zum Beispiel AF-Heparin-Toyopearl® (ein synthetisches, grossporiges, hydrophiles Polymer auf der Basis von Methacrylat (Tosohaas), Heparin EMD-Fraktogel® (ein synthetisches, hydrophiles Polymer auf der Basis von Ethylenglykol, Methacrylat und Dimethylacrylat) (Merck) oder Heparin Sepharose Fast Flow® (enthaltend natürliche Dextran- bzw. Agarosederivate) (Pharmacia).

Die Affinitätschromatographie wird vorzugsweise in einem pH-Bereich von 6,0 bis 8,5, und insbesondere bei einem pH-Wert von 7,4, durchgeführt.

Im erfindungsgemässen Verfahren wird als Puffersystem eine Pufferlösung bestehend aus Puffersubstanzen, insbesondere Tris/HCl-Puffer, Phosphatpuffer oder Citratpuffer, und gegebenenfalls Salz verwendet, die vorzugsweise frei von Stabilisatoren, Aminosäuren und anderen Zusätzen ist. Es hat sich gezeigt, dass bei einer mit EDTA behandelten vWF-Lösung in einem Ca²⁺-freien Puffersystem eine besonders gute Trennung von niedermolekularen und hochmolekularen vWF-Proteinaggregaten erzielt werden kann. Auf diese Weise lassen sich deshalb auch die hochmolekularen vWF-Fraktionen oder hochmolekularen rvWF-Fraktionen, denen eine besonders hohe physiologische Aktivität zukommt, mit guten Ausbeuten erhalten.

Bei dem erfindungsgemässen Verfahren wird vorzugsweise ein rekombinantes vWF-Konzentrat aus zellfreien Kulturüberständen transformierter Zellen eingesetzt.

Nach dem erfindungsgemässen Verfahren zur Trennung von vWF in hochmolekulare und niedermolekulare Fraktionen lassen sich auf effiziente und einfache Weise niedermolekularer und hochmolekularer vWF erhalten. Nach diesem Trennverfahren lässt sich deshalb die physiologisch besonders aktive und somit zur Behandlung von Hämophilie A und verschiedener Formen der von Willebrand Disease hervorragend geeignete hochmolekulare oder niedermolekulare Fraktion von vWF mit guten Ausbeuten herstellen.

In einer bevorzugten Ausführungsform gemäss der vorliegenden Erfindung wird gereinigter vWF in hochmolekularen vWF und niedermolekularen vWF aufgetrennt.

Der für die Trennung eingesetzte vWF kann mit Hilfe jedes bekannten Verfahrens vorgereinigt werden.

Besonders bevorzugt ist der gereinigte von Willebrand-Faktor nach einem Verfahren erhältlich, welches die Stufen umfasst, dass man plasmatischen von Willebrand-Faktor durch eine Anionenaustauschchromatographie an einem Anionenaustauscher vom quaternären Aminotyp und eine Affinitätschromatographie an immobilisiertem Heparin in einer Pufferlösung bestehend aus Puffersubstanzen und ggf. Salz chromatographisch reinigt.

In einer weiteren bevorzugten Ausführungsform ist der gereinigte von Willebrand-Faktor nach einem Verfahren erhältlich, welches die Stufen umfasst, dass man rekombinanten von Willebrand-Faktor durch eine Anionenaustauschchromatographie an einem Anionenaustauscher vom quaternären Aminotyp und eine Affinitätschromatographie an immobilisiertem Heparin in einer Pufferlösung bestehend aus Puffersubstanzen und ggf. Kochsalz chromatographisch reinigt.

Besonders bevorzugt ist der gereinigte von Willebrand-Faktor nach einem Verfahren erhältlich, bei dem ein rvWF-Konzentrat aus zellfreien Kulturüberständen transformierter Zellen gereinigt wird.

Als Pufferlösung wird bei der Reinigung von vWF bevorzugt ein von Stabilisatoren, Aminosäuren und anderen Zusätzen freies Puffersystem verwendet.

Bevorzugt wird die Anionenaustauschchromatographie und/oder die Affinitätschromatographie in einem pH-Bereich von 6,0 bis 8,5, und bevorzugter bei einem pH-Wert von 7,4, durchgeführt.

Der bei der Anionenaustauschchromatographie an den Anionenaustauscher und bei der Affinitätschromatographie an immobilisiertes Heparin gebundene von Willebrand-Faktor kann durch Erhöhung der Salzkonzentration eluiert werden.

Bevorzugt ist der quaternäre Anionenaustauscher ein Fraktogel mit Tentakelstruktur, insbesondere ein EMD-TMAE-Fraktogel.

Bei dem Reinigungsverfahren wird insbesondere der vWF an den Anionenaustauscher bei einer Salzkonzentration von≤ 270 mM NaCl gebunden, und bei einer Salzkonzentration > 270 mM NaCl, und vorzugsweise > 280 mM NaCl eluiert.

Die Affinitätschromatographie wird bei der Reinigung des vWF vorzugsweise an einem Träger mit daran gebundenem Heparin durchgeführt, wobei sich bevorzugt AF-Heparin-Toyopearl® (Tosohaas), Heparin EMD-Fraktogel® (Merck) und Heparin Sepharose Fast Flow® (Pharmacia) gleichermassen eignen.

In einer besonderen Ausführungsform ist der gereinigte von Willebrand-Faktor nach einem Verfahren erhältlich, bei dem der in der Anionenaustauschchromatographie vorgereinigte vWF an das immobilisierte Heparin bei einer Salzkonzentration < 150 mM NaCl bindet und bei einer Salzkonzentration > 150 mM NaCl, vorzugsweise bei 200 bis 300 mM NaCl, bevorzugter 160 bis 270 mM NaCl eluiert wird.

Es ist von Vorteil, als Ausgangsmaterial zur Trennung von hochmolekularem und niedermolekularem vWF bereits gereinigte Konzentrate heranzuziehen. Das chromatographische Trennungsverfahren bewirkt noch zusätzlich einen weiteren Reinigungseffekt.

Die nach dem erfindungsgemässen Trennungsverfahren erhaltenen hochmolekularen und niedermolekularen Fraktionen sowie Mischungen der beiden Fraktionen in jedem Mischungsverhältnis sind physiologisch aktiv und können für therapeutische Zwecke eingesetzt werden.

Die niedermolekulare Fraktion an vWF-Molekülen ist dadurch gekennzeichnet, dass sie vorwiegend Dimere und Tetramere enthält, die aus identischen vWF-Untereinheiten bestehen.

In der niedermolekularen Fraktion ist der Anteil an Dimeren und Tetrameren höher als in der physiologischen Zusammensetzung des vWF.

Es wurde überraschenderweise gefunden, dass niedermolekulare Fraktionen von rekombinantem aus identischen vWF-Untereinheiten bestehen, wogegen Fraktionen von plasmatischem vWF aus einer Mischung von Dimeren oder Tetrameren mit unterschiedlichen Untereinheiten bestehen.

Die niedermolekulare Fraktion an vWF-Molekülen ist nach einem Verfahren gemäss einem der Ansprüche 1 bis 13 erhältlich.

Die niedernnolekulare Fraktion von vWP-Molekülen enthält mindestens 83 % Dimere und maximal 16 % Tetramere und maximal 1 % höhere Polymere. Die niedermolekulare Fraktion ist frei von Plättchen aggregierender Wirkung, bindet Faktor VIII, trägt zur Stabilisierung von Faktor VIII bei und beeinflusst die Lagerstabilität von Faktor VIII positiv.

Die niedermolekulare Fraktion von vWF-Molekülen besteht vorzugsweise aus rekombinanten vWF.

Die hochmolekulare Fraktion von vWF-Molekülen, weist eine um mindestens 50 %, vorzugsweise mindestens 60 % verbesserte Aktivität pro µg Protein in der Plättchenaggregation verglichen mit der physiologischen Mischung von hochmolekularen und niedermolekularen vWF-Molekülen auf.

Die hochmolekulare Fraktion von vWF-Molekülen ist nach einem Verfahren gemäss einem der Ansprüche 1 bis 13 erhältlich.

Überaschenderweise wurde gefunden, dass die hochmolekulare Fraktion von rekombinantem vWF Multimere enthält, die aus identischen vWF-Untereinheiten bestehen, wogegen Fraktionen von plasmatischem vWF aus einer Mischung von Multimeren mit unterschiedlichen Untereinheiten bestehen.

Überraschenderweise wurde gefunden, dass sowohl die niedermolekulare als auch die hochmolekulare Fraktion von rekombinantem vWF eine potentiell höhere Bindungskapazität an Faktor VIII im Vergleich zu entsprechenden Fraktionen des plasmatischen vWF besitzt. Die Fraktionen des rekombinanten vWF binden daher Faktor VIII effizienter als die plasmatischen Fraktionen.

Die unterschiedliche Verwendung der hochmolekularen bzw. der niedermolekularen Fraktion ergibt sich aus ihrer physiologischen Aktivität. Die Möglichkeit der Herstellung von gezielten Mischungen der beiden Fraktionen erlaubt die Behandlung von speziellen medizinischen Indikationen.

Weiterer Gegenstand der vorliegenden Erfindung ist somit die Verwendung der niedermolekularen Fraktion von vWF-Molekülen oder der hochmolekularen Fraktion von vWF-Molekülen gemäß den Ansprüchen zur Behandlung von Hämophilie A oder verschiedener Formen der von Willebrand Disease.

Insbesondere sei darauf hingewiesen, dass bei Verwendung eines rekombinanten vWF in dem erfindungsgemässen Trennungsverfahren die Endprodukte (hochmolekulare und niedermolekulare Fraktion, sowie Mischungen der beiden Fraktionen in jedem Mischungsverhältnis) frei von Plasmaproteinen und frei von Faktor VIII sowie frei von pathogenen Viren sind. Dieses kann bei der Herstellung von pharmazeutischen Produkten, wie sie oben genannt sind, für bestimmte medizinische Indikationen von grossem Vorteil sein.

Gegenstand der vorliegenden Erfindung ist auch eine medizinische Zusammensetzung, die die niedermolekulare Fraktion von vWF-Molekülen oder die hochmolekulare Fraktion von vWF-Molekülen gemäß den Ansprüchen in einem physiologisch annehmbaren Träger enthält.

Vorzugsweise enthält die pharmazeutische Zusammensetzung nach den Ansprüchen Faktor VIII oder funktionelle Deletionsmutante(n) von Faktor VIII, wobei die vWF-Moleküle der hochmolekularen Fraktion oder der niedermolekularen Fraktion oder der Mischung davon Faktor VIII oder funktionelle Deletionsmutante(n) von Faktor VIII stabilisieren.

Zur Herstellung pharmazeutischer Präparationen werden die jeweiligen Fraktionen oder deren Mischungen vorzugsweise aufkonzentriert und das Konzentrat weiter verarbeitet.

Die Herstellung der pharmazeutischen Zusammensetzungen kann auf an sich bekannte und übliche Weise erfolgen. Vorzugsweise werden die Produkte (niedermolekulare und hochmolekulare Fraktionen) oder die diese enthaltenden Konzentrate mit einem geeigneten physiologisch verträglichen Träger vermischt. Als Träger dient vorzugsweise eine physiologische Kochsalzlösung.

Die pharmazeutischen Zusammensetzungen können in einer zur Behandlung von Hämophilie A und verschiedener Formen der von Willebrand Disease üblichen und gebräuchlichen Darreichungsform vorliegen; vorzugsweise liegen sie in Form eines zur Infusion geeigneten Präparates vor:

In den folgenden Beispielen wird die Erfindung näher erläutert, ohne sie darauf zu beschränken.

In Beispiel 1 wird die erfindungsgemässe Trennung von hochmolekularen und niedermolekularen vWF-Polymeren mittels Heparin-Affinitätschromatographie gezeigt.

### Beispiel 1: Trennung von hoch- und niedermolekularen Formen des von Willebrand-Faktors

### Rekombinanter von Willebrand-Faktor (rvWF) in 20 mM Tris-HCl

Puffer (Tris-Puffer), pH 7,4, wurde auf eine Glassäule, welche mit AF Heparin Toyopearl® (Firma Tosohaas) gefüllt war, mit einer Fliessgeschwindigkeit von 1 ml/min/cm² aufgetragen. Die Säule wurde zuerst mit Tris-Puffer gespült, um unspezifisch gebundene Proteine zu entfernen. Dann wurde die Säule mit 160 mM NaCl in Tris-Puffer und nachfolgend mit 270 mM NaCl in Tris-Puffer eluiert. Während der Chromatographie wurde die Proteinabsorbtion in üblicher Weise bei 280 nm verfolgt. Nach der Chromatographie wurde die Proteinkonzentration mittels der Bradford Methode (M. Bradford, Anal. Biochem. 72:248-254, 1976) bestimmt. Der Gehalt an rvWF wurde mittels eines handelsüblichen ELISA Systems (Boehringer Mannheim) bestimmt. Die Verteilung der Multimerenstrukturen des rekombinanten von Willebrand-Faktors in den einzelnen Reinigungsfraktionen wurde durch SDS-Agarosegelelektrophorese in üblicher Weise untersucht und durch Densitometrie quantitativ ausgewertet. Die Fähigkeit zur Ristocetin vermittelten Blutplättchenaggregation wurde mittels eines handelsüblichen Testsystems (von Willebrand Reagens, Behring Werke) untersucht.

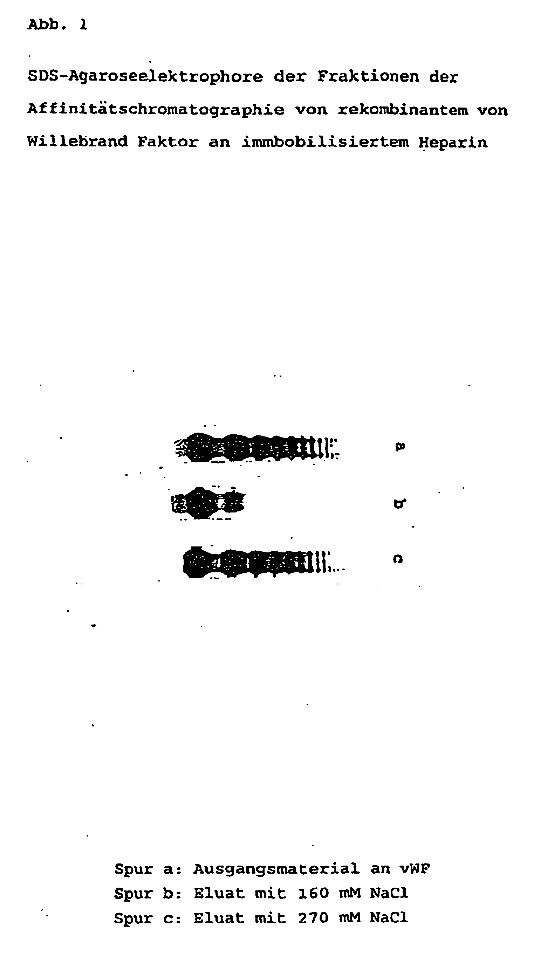

Abbildung 1 zeigt das Multimerenspektrum der einzelnen erhaltenen Fraktionen. Bei einer Salzkonzentration von 160 mM NaCl wurde rvWF mit niedrigem Molekulargewicht, vorwiegend vom Typ primäres Dimer, eluiert. Demgegenüber wurde bei einer Salzkonzentration von 270 mM NaCl rvWF mit hohem Molekulargewicht eluiert.

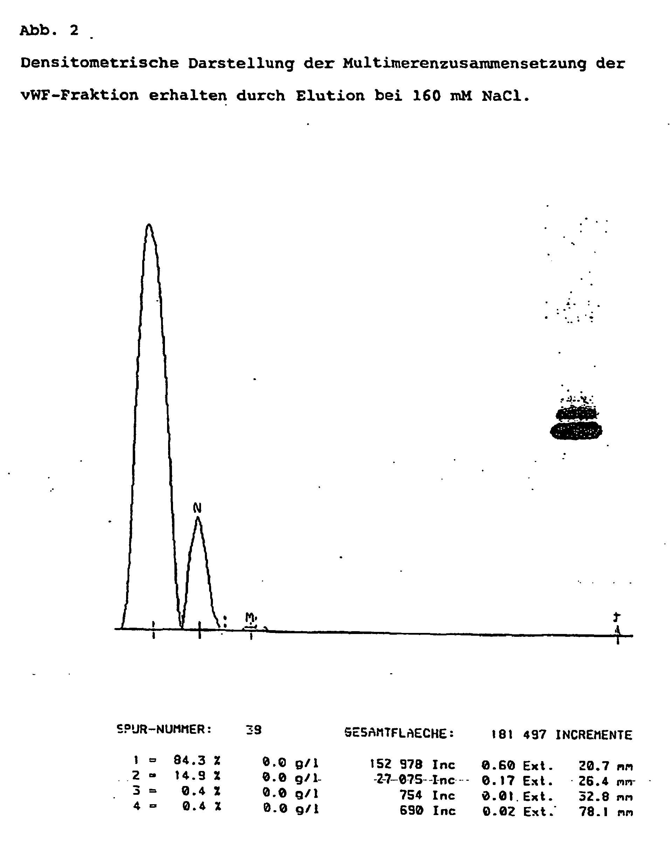

Abbildung 2 zeigt eine densitometrische Auswertung der Verteilung der Multimeren des bei 160 mM NaCl erhaltenen rekombinanten von Willebrand-Faktors. Diese Auswertung ergab, dass das so erhaltene rvWF-Polymergemisch zu 84,4 % aus dem primären Dimeren (Molekulargewicht ca. 440000) und zu 14,9 % aus dem Tetrameren (Molekulargewicht ca. 880000) besteht.

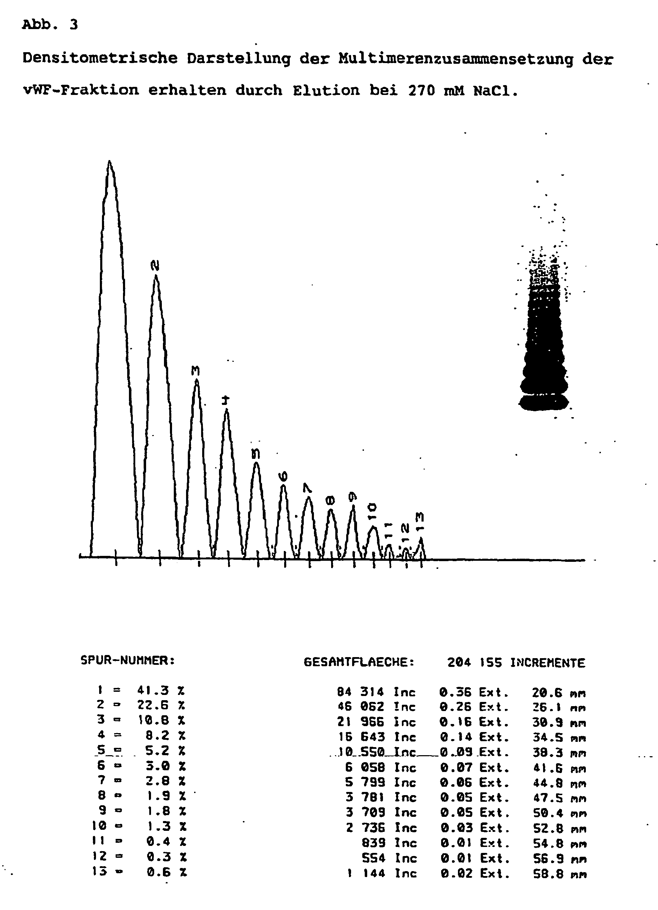

Eine in Abbildung 3 gezeigte densitometrische Auswertung der rvWF Fraktion, die durch Elution mit 270 mM NaCl erhalten wurde, ergab, dass das so erhaltene rvWF-Polymergemisch aus einer Kaskade von immer höhermolekularen Polymeren besteht.

In Tabelle 1 sind die Ergebnisse der Chromatographie und erste Funktionsanalysen der rvWF Fraktionen zusammengestellt. Aus den Ergebnissen geht hervor, dass nahezu der gesamte rvWF an den Träger gebunden wurde. Das mit 160 mM NaCl eluierte rvWF-Polymergemisch (niedermolekular) zeigte keine Fähigkeit zur Agglutination von Blutplättchen. Die bei 270 mM NaCl erhaltene Fraktion (hochmolekular) besass eine hohe Aktivität in Bezug auf Plättchen-Aggregation. Gegenüber dem Ausgangsmaterial wurde dabei durch die Abtrennung der nicht aggregierenden, niedermolekularen rvWF Formen die spezifische Aktivität in Bezug auf die Agglutination um 69 % gesteigert.

Durch die im Beispiel 1 beschriebene Chromatographie des rvWF am Affinitätsträger wurde gleichzeitig die Reinheit des rvWF wesentlich erhöht. rvWF der 160 mM NaCl-Fraktion zeigte eine doppelte, und rvWF der 270 mM NaCl Fraktion eine 6 fach höhere Reinheit im Vergleich zum Ausgangsmaterial.

**Tabelle 1**

| Probe | Volumen | Protein | vWF:AG | vWF:AG | mg vWF:AG/ | Agglutination | Agglutination |
|---|---|---|---|---|---|---|---|
| | ml | mg/ml | mg/ml | mg | mg Protein | mU/µg Protein | mU/µg vWF:AG |
| rvWF Ausgang | 560 | 0,62 | 0,92 | 51 | 0,15 | 0,650 | 4,3 |
| | | | | | | | |
| 160 mM NaCl-Eluat | 98 | 0,2 | 0,54 | 6 | 0,27 | 0 | 0 |
| | | | | | | | |
| 270 mM NaCl-Eluat | 86 | 0,5 | 0,440 | 38 | 0,88 | 6,4 | 7,27 |

### Beispiel 2: Bindung von rekombinantem von Willebrand-Faktor an Faktor VIII

Die nachfolgenden Untersuchungen wurden in ELISA-Mikrotitrationsplatten durchgeführt, die mit anti vWF Immunglobulin (Asserachrom vWF, Boehringer Mannheim) beschichtet waren. Durch 1-stündige Inkubation bei Raumtemperatur mit 200 µl 50 mM Tris-HCl pH 7,4, 150 mM NaCl, 0,5 % Albumin, 0,1 % Tween 20 (TBS-Puffer), der 100 ng/ml vWF:AG enthielt, wurden jeweils 5 ng vWF an die Reaktionsgefässe gekoppelt. Es wurden folgende vWF Proben verwendet: Humaner vWF (Diagnostika Stago), rvWF (Ausgangsmaterial aus Beispiel 1), niedermolekularer rvWF (160 mM NaCl Elutions-Fraktion aus Beispiel 1), hochmolekularer rvWF (270 mM NaCl Elutions-Fraktion aus Beispiel 1). Die Mikrotitrationsplatten wurden anschliessend 3 mal für 3 Minuten mit TBS gewaschen, und anschliessend wurde jedes Reaktionsgefäss mit 25 µl rekombinantem Faktor VIII (Miles) in TBS überschichtet und 1 Stunde bei Raumtemperatur inkubiert. Die Konzentration des Faktor VIII wurde im Bereich von 100 mU/ml bis 7,12 mU/ml variiert. Danach wurden die Reaktionsgefäss 3 mal für 3 Minuten mit jeweils 200 µl TBS gewaschen. Anschliessend wurde die durch den immobilisierten vWF gebundene Menge an Faktor VIII mittels eines käuflichen Testsystems (Chromogenix Coatest VIII:C/4) bestimmt.

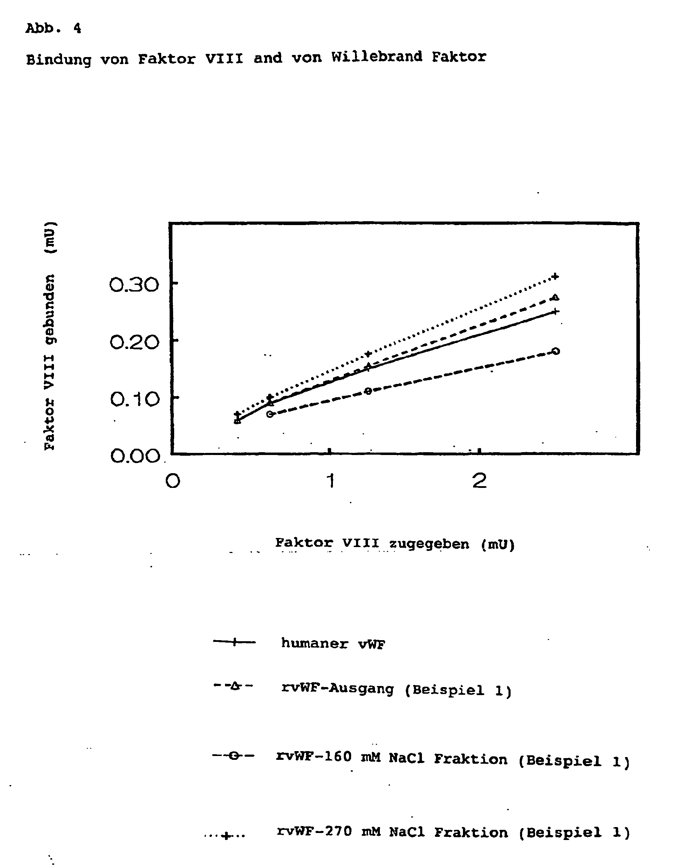

Abbildung 4 zeigt die Bindung von Faktor VIII an den von Willebrand-Faktor in Abhängigkeit von der angebotenen Faktor VIII Menge. Die Ergebnisse zeigen, dass alle untersuchten vWF Fraktionen den Faktor VIII in einer konzentrationsabhängigen Weise binden, wobei rvWF verglichen mit humanem vWF eine annähernd identische Faktor VIII Bindung zeigte. rvWF der 270 mM NaCl. Fraktion aus Beispiel 1 zeigte eine erhöhte Bindung von Faktor VIII, rvWF aus der 160 mM NaCl Fraktion eine geringfügig niedrigere Bindung von Faktor VIII.

### Beispiel 3: Stabilisierung von rekombinantem Faktor VIII durch rekombinanten von Willebrand-Faktor.

Faktor VIII wird physiologisch im menschlichen Körper durch den von Willebrand-Faktor gebunden und ist in dieser Form stabil. Demgegenüber wird ungebundener Faktor VIII in wenigen Minuten proteolytisch inaktiviert.

Unterschiedliche Fraktionen von rekombinantem von Willebrand-Faktor, erhalten nach der Methode aus Beispiel 1, wurden transformierten SK-Hep Zellen in Zellkultur zugesetzt, die Faktor VIII sezernieren. Nach 24 Stunden wurden in den Zellkultur-Überständen die Aktivitäten von rekombinantem Faktor VIII mittels eines käuflichen Testsystems (Chromogenix Coatest VIII:C/4) bestimmt. Tabelle 2 stellt die Ergebnisse zusammen.

Aus den Ergebnissen ist ersichtlich, dass durch die Zugabe von rvWF zu den Zellen nach 24 Stunden eine wesentlich höhere Konzentration an Faktor VIII vorhanden war. Sowohl die hochmolekulare vWF Fraktion als auch die niedermolekulare vWF Fraktion binden und stabilisieren den Faktor VIII, wobei die hochmolekulare Fraktion eine bessere Fähigkeit zur Stabilisierung von Faktor VIII hat als die niedermolekulare Fraktion.

Diese Daten zeigen ausserdem, dass durch Zugabe von von Willebrand-Faktor auch rekombinant hergestellter Faktor VIII stabilisiert werden kann.

**Tabelle 2:**

| **Probe 15 µg/ml** | **Faktor VIII Aktivität nach 24 Stunden U/ml** |
|---|---|
| Kontrolle | 0,5 |
| rvWF Ausgang | 1,8 |
| rvWF-160 mM NaCl Eluat | 0,8 |
| rvWF-270 mM NaCl Eluat | 2,4 |

### Beispiel 4: Stabilisierung von Faktor VIII durch rekombinanten von Willebrand-Faktor

Gemische aus Faktor VIII und rekombinantem von Willebrand-Faktor aus Zellkulturüberständen, wie sie im Beispiel 3 erhalten wurden, wurden bei -20°C eingefroren und für 6 Tage bei -20°C aufbewahrt. Danach wurde wiederum die Aktivität an Faktor VIII bestimmt. Tabelle 3 fasst die Ergebnisse zusammen.

Aus den Ergebnissen ist ersichtlich, dass alle getesteten vWF Fraktionen die Lagerstabilität des Faktors VIII um mehr als das 2,5fache erhöhen. Dieses lässt auch bei der niedermolekularen Fraktion auf eine starke Bindung des vWF-Faktor VIII Komplexes schliessen.

**Tabelle 3**

| **Probe 15 µg/ml** | **Faktor VIII Aktivität (mU/ml)** | | |
|---|---|---|---|
| | **t=0** | **t=6 Tage** | **%Verlust** |
| Kontrolle | 500 | 150 | 70 |
| rvWF Ausgang | 1800 | 1300 | 28 |
| rvWF-160 mM NaCl Eluat | 800 | 600 | 25 |
| rvWF-270 mM NaCl Eluat | 2400 | 1800 | 25 |

### Beispiel 5: Reinigung von rvWF aus Kulturüberständen durch Anionenaustauschchromatographie

Rekombinanter vWF wurde gemäss üblicher Verfahren nach Infektion von Vero Zellen (Affen-Nierenzellen) mit Vaccinia-Virus in Zellkulturtechnik gewonnen. Vero/Vaccinia Expressionssysteme und Zellkulturbedingungen werden in F.G. Falkner et al., Thrombosis and Haemostasis 68 (1992) 119-124, N. Barrett et al., AIDS Res. 5 (1989) 159-171 und F. Dorner et al., AIDS Vaccine Research and Clinical Trials, Marcel Dekker, Inc, New York (1990) ausführlich beschrieben. Die Expression von rvWF erfolgte in synthetischem DMEM-Standardmedium (Dulbeccos minimal essential medium).

Nach der Fermentation der transformierten Zellen wurde das Kulturmedium abgetrennt, und Zellen und Zellbruchstücke wurden durch Zentrifugation entfernt. Weitere kleinere Bestandteile, wie Membranbruchstücke oder Bakterien, wurden durch Filtration durch Filter mit einer Porengrösse von 0,4 µm entfernt.

770 ml zellfreier Kulturüberstand wurde mit einer Fliessgeschwindigkeit von 2 ml/cm2/min über eine Säule (1,6 cm x 5 cm, gefüllt mit 10 ml Anionenaustauscher EMD-TMAE-Fraktogel (Merck)) filtriert. Das Gel wurde zuvor mit 20 mM Tris-HCl Puffer (pH 7,4) equilibriert. Anschliessend wurde die Säule mit 20 mM Tris-HCl Puffer (pH 7,4) gewaschen. Fremdstoffe wurden durch Waschen der Säule mit 200 mM NaCl enthaltendem Puffer entfernt. Der rvWF wurde dann mit 280 mM NaCl in 20 mM Tris-HCl Puffer (pH 7,4) vom Träger eluiert. Schliesslich wurde mit 1 M NaCl eventuell vorhandenes Restmaterial von der Säule eluiert. Während der Chromatographie wurde die Proteinabsorption in üblicher Weise bei 280 nm verfolgt. Nach der Chromatographie wurde die Proteinkonzentration nach der Bradford Methode (M. Bradford, Anal. Biochem. 72 (1976) 248-254) bestimmt. Der Gehalt an rvWF wurde mittels eines handelsüblichen ELISA Systems (Boehringer Mannheim) bestimmt.

Es wurde gefunden, dass nahezu der gesamte rvWF an den Träger gebunden wurde. rvWF wurde durch 0,28 M NaCl vom Anionenaustauscher eluiert. Die Ergebnisse der Reinigung von rvWF am Anionenaustauscher sind in Tabelle 4 zusammengefasst.

Durch die in diesem Beispiel beschriebene Reinigung wurde der rvWF um das 6-fache angereichert.

**Tabelle 4**

| **Probe** | **Volumen (ml)** | **Gesamtprotein (µg/ml)** | **rvWF (µg/ml)** | **rvWF/ Gesamtprotein** |
|---|---|---|---|---|
| Zellfreier Kulturüberstand | 770 | 113 | 7,9 | 0,069 |
| Elution mit 200 mM NaCl | 95 | 147 | 0,0016 | 0,00001 |
| Elution mit 280 mM NaCl | 75 | 168 | 61 | 0,36 |
| Elution mit 1 M NaCl | 50 | 196 | 6 | 0,03 |

### Beispiel 6: Reinigung von rvWF durch Affinitätschromatographie

Ein nach Beispiel 5 erhaltener rvWF wurde zur Verringerung der Salzkonzentration (160 mM NaCl) mit 20 mM Tris-HCl Puffer (pH 7,4) verdünnt. Anschliessend wurde die Lösung durch eine Säule (1,6 cm x 5 cm, gefüllt mit 10 ml AF Heparin Toyopearl 650 (Tosohaas)) mit einer Fliessgeschwindigkeit von 1 ml/cm2/min filtriert. Die Säule war zuvor mit 20 mM Tris-HCl Puffer (pH 7,4) equilibriert worden. Unspezifisch gebundene Proteine wurden zuerst durch Waschen mit 20 mM Tris-HCl Puffer (pH 7,4) entfernt. rvWF wurde durch 270 mM NaCl in 20 mM Tris-HCl Puffer (pH 7,4) vom Träger eluiert. Schliesslich wurde mit 1 M NaCl Restmaterial von der Säule eluiert. Während der Chromatographie wurde die Proteinabsorbtion in üblicher Weise bei 280 nm verfolgt. Nach der Chromatographie wurde die Proteinkonzentration mittels der Bradford Methode (M. Bradford, l.c.) bestimmt. Der Gehalt an rvWF wurde mittels eines handelsüblichen ELISA Systems (Boehringer Mannheim) bestimmt.

Es wurde gefunden, dass nahezu der gesamte rvWF an den Träger gebunden wurde. Bei der Elution mit 270 mM NaCl wurde der Grossteil des rvWF von der Säule eluiert, während die Waschung mit 1 M NaCl nur noch Spuren von rvWF enthält. Die Ergebnisse dieses Reinigungsschrittes sind in Tabelle 5 zusammengefasst. Durch diesen Reinigungsschritt wurde der Anteil des rvWF-Proteins zum Gesamtprotein auf über 86 % erhöht.

Mit einer denaturierenden SDS-Proteingelelektrophorese (U.K. Laemmli, Nature 227 (1970) 680-685) und anschliessendem Western-Blot wurde die Fraktion von 270 mM NaCl genauer untersucht.

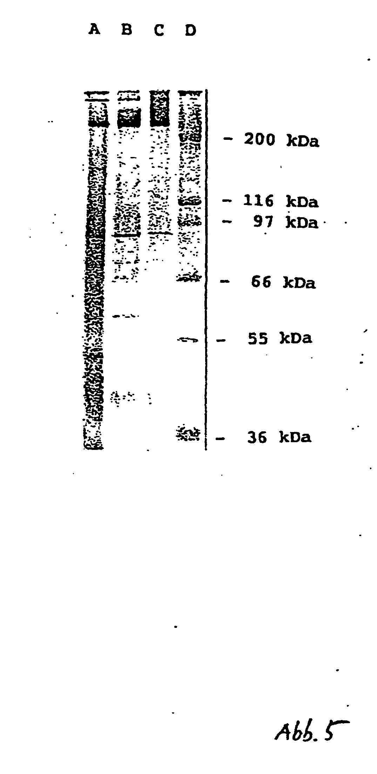

Wie in Abb. 5 dargestellt, ergab die denaturierende elektrophoretische Analyse, dass durch die im Beispiel 5 und 6 beschriebene Reinigung rvWF in hoher Reinheit gewonnen wurde. Im so gewonnenen Produkt konnten keine anderen Gerinnungsfaktoren, wie z.B. Faktor VIII, nachgewiesen werden.

**Tabelle 5:**

| **Probe** | **Volumen (ml)** | **Gesamtprotein (µg/ml)** | **rvWP (µg/ml)** | **rvWF/ Gesamtprotein** |
|---|---|---|---|---|
| rvWF-Konzentrat | 225 | 50 | 13,9 | 0,27 |
| Elution mit 270 mM NaCl | 43 | 70 | 60 | 0,86 |
| Elution mit 1 M NaCl | 32 | 25 | 2 | 0,08 |

### Beispiel 7: Isolierung von plasmatischem und rekombinantem von Willebrand-Faktor mit unterschiedlicher Multimerisierung und Charakterisierung der Bindung zu Faktor VIII

Rekombinanter von Willebrand-Faktor (r-vWF), von Willebrand-Faktor aus Humanplasma (p-vWF) und von Willebrand-Faktor aus Plasma-Kryopräzipitat (k-vWF) wurde durch Kombination aus Anionen-Austauschchromatographie und Heparin-Affinitätschromatographie gereinigt.

In der Anion-Austauschchromatographie wurde das Ausgangsmaterial (r-vWF: Fermentationsüberstand rekombinanter CHO-Zellen; p-vWF: humanes Citratplamsa; k-vWF: Plasmakryopräzipitat) auf eine Fractogel-EMD-TMAE-Säule (Firma Merck) aufgetragen und vWF durch Elution mit 20 mM Tris/HCl-Puffer, pH 7,0, 280 mM NaCl, erhalten. Anschliessend wurden die Präparate durch Zugabe von 20 mM Tris/HCl-Puffer auf eine Salzkonzentration von 90 mM verdünnt und auf eine Fractogel-EMD-Heparin-Säule (Fa. Merck) aufgetragen. vWF wurde durch eine stufenweise Elution mit zunehmender Salzkonzentration eluiert. Dabei wurde die NaCl-Konzentration in 20 mM Tris/HCl-Puffer, pH 7,0, zwischen 120 mM und 280 mM variiert. Auf diese Weise wurden sowohl für p-vWF, k-vWF und r-vWF Präparate bei 120 mM NaCl, 160 mM NaCl, 190 mM NaCl, 230 mM NaCl und 280 mM NaCl erhalten, die sich in der Multimerenzusammensetzung voneinander unterscheiden.

Der Gehalt an vWF (vWF-Antigen, vWF:Ag) wurde mittels einem üblichen ELISA-Test bestimmt (Asserachrom vWF, Boehringer Mannheim).

Die Bindung von Gerinnungsfaktor VIII an sowohl p-vWF, k-vWF und r-vWF wurde mittels 'real-time biospecific interaction analysis', basierend auf Messungen der Oberflächen-Plasmon Resonanz-Technologie (Malmqvist M, Natur 1993; 361: 186-187) bestimmt. Dabei wurde das stöchiometrische Verhältnis von vWF-Untereinheiten zu gebundenem Faktor VIII bestimmt: Ein monoklonaler antihuman von Willebrand-Antikörper (AvW82) wurde kovalent an den Sensorchip CM5 (Pharmacia Biosensor AB) gebunden (O'Shannessy D.J. et al, 1993, Anal. Biochem. 212: 457-468; Karlsson R, 1994, Anal. Biochem. 221: 142-151). Die Messung der Resonanz-Einheiten (RU) entspricht dabei der Basislinie (RU_{BL}).

p-vWF, k-vWF und r-vWF wurden in 10 mM HEPES, pH 7,4, 105 mM NaCl, 1 mM CaCl₂, 0,05% Surfactant P 20 (HBS-Puffer) zu einer Konzentration von 20µg/ml gelöst. Für jedes Experiment wurden 50µl Proben des vWF über den Sensorchip aufgetragen, mit einer Fliessgeschwindigkeit von 5µl/min, um die Bindung von AvW8/2 an den vWF zu ermöglichen (Phase A).Nicht gebundener vWF wird durch Spülen mit HBS-Puffer entfernt (Phase B). p-vWF und k-vWF wurden zusätzlich mit 20µl 250 mM CaCl₂ gewaschen , um Spuren von plasmatischem Faktor VIII zu entfernen. Die Bindung von vWF an Av8/2 entspricht der vWF-Resonanz-Einheit (RU_{vWF}). Für die Analyse der Bindung des Faktors VIII wurden 60 ml rekombinanter FVIII (2,5 µg/ml in HBS-Puffer) über den Sensorchip injiziert, bei konstanter Flussrate von 5µl/ml, um die Bindung des Faktors VIII an den vWF zu ermöglichen (Phase C). Die Messung der Resonanzeinheiten entsprechen der FVIII-Bindung (RU_{FVIII}). Durch Spülen mit HBS-Puffer wurde Faktor VIII wieder vom vWF abdissoziiert (Phase D). Das stöchiometrische Verhältnis des Komplexes aus FVIII und vWF-Untereinheit ergibt sich aus den Resonanzeinheiten und den Molekulargewichten: vWF (Untereinheit) : FVIII = (RU_{vWF} - RU_{BL}) / (RU_{FVIII} - RU_{vWF}) x 330,000/220,000 (O'Shannessy D J et al, 1993, Anal. Biochem. 212: 457-468; Karlsson R, 1994, Anal. Biochem. 221: 142-151).

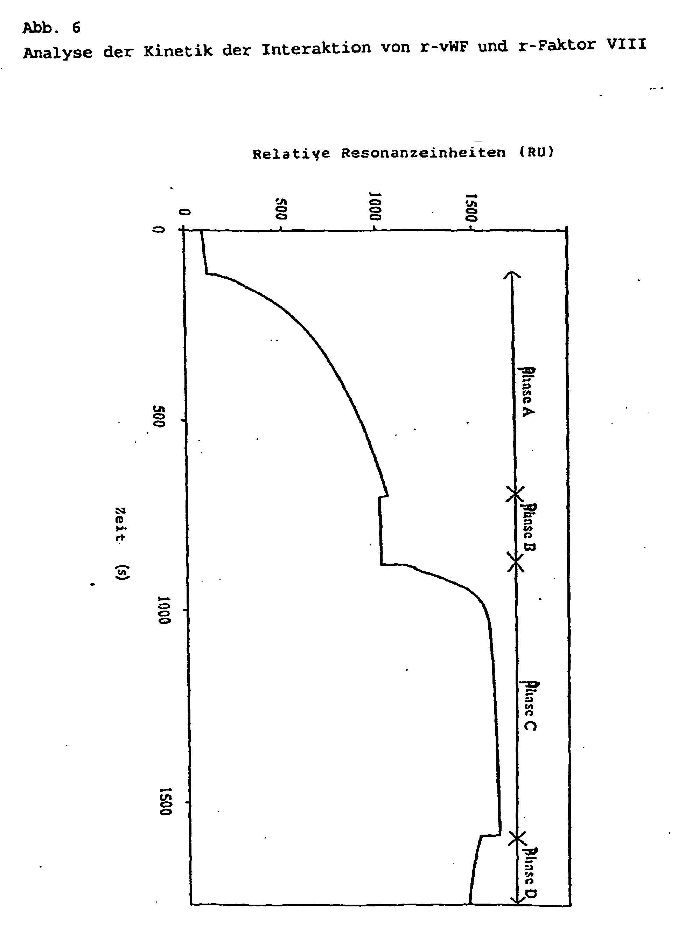

Abb. 6 zeigt das Sensogramm der Anlagerung von r-Faktor VIII an r-vWF.

**Tabelle 6**

| Bestimmung der Stöchiometrie des vWF-Faktor VIII-Komplexes | |
|---|---|
| Beispiel | Stöchiometrie vWF-Untereinheit : FVIII |
| r-vWF 120 mM Eluat | 2,25 : 1 |
| r-vWF 160 mM Eluat | 2,5 : 1 |
| r-vWF 190 mM Eluat | 2,0 : 1 |
| r-vWF 230 mM Eluat | 2,0 : 1 |
| r-vWF 280 mM Eluat | 2,0 : 1 |
| k-vWF 280 mM Eluat | 2,6 : 1 |
| P-VWF 280 mM Eluat | 3 : 1 |

Tabelle 6 zeigt die Stöchiometrie von vWF : : Faktor VIII von nieder- und hochmolekularen Fraktionen von plasmatischem und rekombinantem vWF. Die Daten zeigen, dass sowohl die niedermolekularen als auch die hochmolekularen Fraktionen von r-vWF eine höhere Bindungskapazität zu Faktor VIII aufweisen als p-vWF.

### Beispiel 8: Reinigung und Trennung von plasmatischem vWF, vWF aus Kryopräzipitat und rekombinantem vWF

Plasmatischer vWF (p-vWF), vWF aus Kryopräzipitat (k-vWF) und rekombinanter vWF (r-vWF) wurden mittels Heparin-Affinitätschromatographie gereinigt und die niedermolekularen und hochmolekularen Fraktionen gemäss Beispiel 1 getrennt.

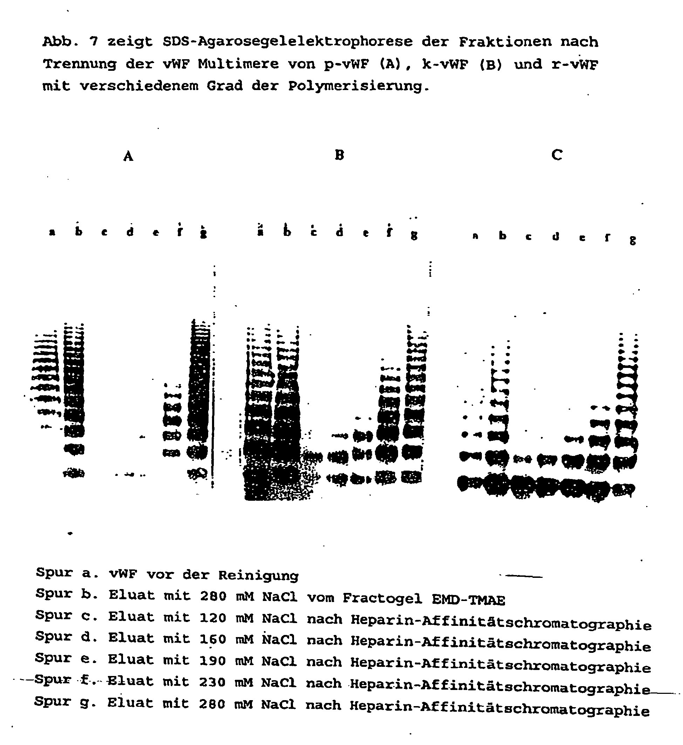

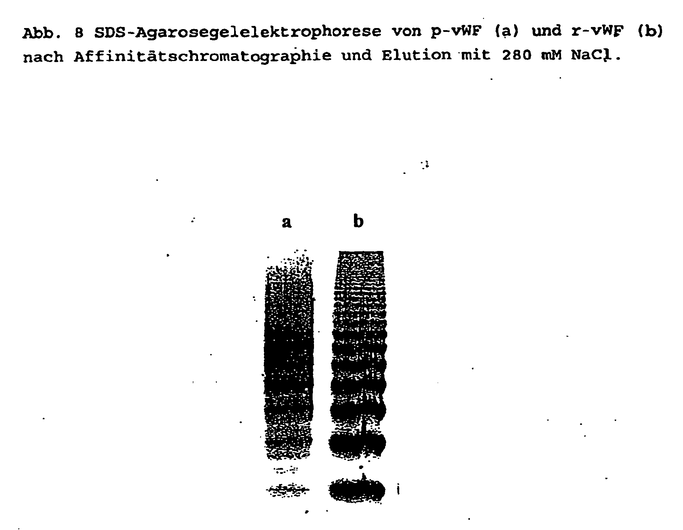

Aus Abb. 7 wird erkennbar, dass die einzelnen Fraktionen von p-vWF und k-vWF wesentlich unschärfere Banden aufweisen als die von r-vWF. Dies wird in Abb. 8 noch wesentlich deutlicher, wo die aufgetrennten Multimere von p-vWF und r-vWF direkt verglichen wurden. Deutlich sichtbar sind die scharfen Banden von r-vWF nach Trennung im SDS-Gel, wogegen bei der Auftrennung von p-vWF eindeutig Zwischenprodukte mit unterschiedlichen Molekulargewichten auftreten. Diese 'Zwischenbanden' sind auf das heterogene Gemisch von verschieden grossen vWF-Untereinheiten in den p-vWF-Fraktionen zurückzuführen, die durch Verdau mit im Plasma vorkommender Protease entstehen. Diese Zwischenprodukte sind in der Literatur als sogenannte Triplet-Strukturen bekannt.

## Patentansprüche

1. Verfahren zur Trennung von vWF in hochmolekularen vWF und niedermolekularen vWF, **dadurch gekennzeichnet, dass** man den vWF an einem Affinitätsträger bindet und dann bei unterschiedlichen Salzkonzentrationen eluiert.

2. Verfahren zur Trennung von vWF nach Anspruch 1, **dadurch gekennzeichnet, dass** der zu trennende vWF in einer an vWF angereicherten Plasmafraktion vorliegt.

3. Verfahren zur Trennung nach Anspruch 1, **dadurch gekennzeichnet, dass** der zu trennende vWF rekombinanter vWF ist, der in einem rekombinanten vWF Konzentrat aus zellfreien Kulturüberständen transformierter Zellen vorliegt.

4. Verfahren zur Trennung von vWF nach Anspruch 1, **dadurch gekennzeichnet, dass** man gereinigten vWF in hochmolekularen vWF und niedermolekularen vWF auftrennt.

5. Verfahren zur Trennung von vWF in hochmolekularen vWF und niedermolekularen vWF nach einem der Ansprüche 1 bis 4, **dadurch gekennzeichnet, dass** man die Trennung in einem Ca²⁺-freien Puffersystem vornimmt.

6. Verfahren zur Trennung von vWF nach einem der Ansprüche 1 bis 5, **dadurch gekennzeichnet, dass** der Affinitätsträger ein Träger mit daran gebundenem Heparin ist, wobei sich bevorzugt AF-Heparin Toyopearl^{®} (Tosohaas), Heparin EVE)-Fraktogel^{®} (Merck) und Heparin Sepharose Fast Flow^{®} (Pharmacia), gleichermassen eignen.

7. Verfahren zur Trennung von vWF nach einem der Ansprüche 1 bis 6, **dadurch gekennzeichnet, dass** als Puffersystem für die Affinitätschromatographie eine Pufferlösung, bestehend aus Puffersubstanzen und ggf. Salz verwendet wird.

8. Verfahren zur Trennung von vWF nach Anspruch 7, **dadurch gekennzeichnet, dass** als Puffersystem eine Pufferlösung, bestehend aus Tris/HCl-Puffer, Phosphatpuffer oder Citratpuffer, und ggf. Kochsalz verwendet wird.

9. Verfahren zur Trennung von vWF nach einem der Ansprüche 1 bis 8, **dadurch gekennzeichnet, dass** die Affinitätschromatographie in einem pH-Bereich von 6,0 bis 8,5, vorzugsweise bei einem pH-Wert von 7,4 durchgeführt wird.

10. Verfahren nach einem der Ansprüche 1 bis 9, **dadurch gekennzeichnet, dass** niedermolekularer vWF bei einer geringeren Salzkonzentration als hochmolekularer vWF eluiert wird.

11. Verfahren nach einem der Ansprüche 1 bis 10, **dadurch gekennzeichnet, dass** man den vWF bei einer Salzkonzentration < 150 mM an den Affinitätsträger bindet, bei einer Salzkonzentration zwischen 150 und 250 mM, vorzugsweise bei 160 mM, niedermolekulare Aggregate des vWF eluiert, und danach bei einer Salzkonzentration von > 250 mM vorzugsweise ≥ 270 mM, hochmolekulare Aggregate des vWF eluiert.

12. Verfahren nach einem der Ansprüche 1 bis 11, **dadurch gekennzeichnet, dass** als Salze lösliche ein- und zweiwertige Salze verwendet werden.

13. Verfahren nach Anspruch 12, **dadurch gekennzeichnet, dass** als Salz NaCl verwendet wird.

14. Zusammensetzung zur medizinischen Verwendung, **dadurch gekennzeichnet, dass** sie eine vWF-Präparation von gereinigten niedermolekularen vWF-Molekülen mit mindestens 83% vWF-Dimeren und maximal 16% vWF-Tetrameren und maximal 1% höheren Polymeren enthält, und einen physiologisch annehmbaren Träger.

15. Zusammensetzung zur medizinischen Verwendung nach Anspruch 14, **dadurch gekennzeichnet, dass** sie rekombinanten vWF enthält, der aus identischen vWF-Untereinheiten besteht.

16. Zusammensetzung zur medizinischen Verwendung nach einem der Ansprüche 14 oder 15, **dadurch gekennzeichnet, dass** die vWF-Präparation frei ist von Plättchen aggregierender Wirkung und Faktor VIII bindet, zur Stabilisierung von Faktor VIII beiträgt, und die Lagerstabilität von Faktor VIII positiv beeinflusst.

17. Zusammensetzung zur medizinischen Verwendung **dadurch gekennzeichnet, dass** die Zusammensetzung:
- eine rekombinante vWF Präparation bestehend aus identischen vWF-Untereinheiten enthält, wobei die rekombinante vWF-Präparation gereinigte hochmolekulare vWF-Moleküle enthält, wobei hochmolekulare vWF-Moleküle solche sind, die eine um mindestens 50%, vorzugsweise mindestens 60% verbesserte Aktivität verglichen mit der physiologischen Mischung von hochmolekularen und niedermolekularen vWF Molekülen pro µg Protein in der Plättchenaggregation, aufweisen, und
- einen physiologisch annehmbaren Träger enthält.

18. Zusammensetzung zur medizinischen Verwendung nach einem der Ansprüche 14 bis 17, **dadurch gekennzeichnet, dass** sie einen Faktor VIII oder funktionelle Deletionsmutante(n) von Faktor VIII enthält.

19. Verwendung einer medizinischen Zusammensetzung nach einem der Ansprüche 14 bis 18, zur Herstellung eines Arzneimittels zur Behandlung von Hämophilie A oder verschiedener Formen der von Willebrand-Disease.

## Claims

1. Process for separating vWF into high-molecular vWF and low-molecular vWF, **characterised in that** the vWF is bound to an affinity support and then eluted at different salt concentrations.

2. Process for separating vWF according to claim 1, **characterised in that** the vWF to be separated is present in a plasma fraction enriched in vWF.

3. Process for separation according to claim 1, **characterised in that** the vWF to be separated is recombinant vWF, which is present in a recombinant vWF concentrate of cell-free culture supernatants of transformed cells.

4. Process for separating vWF according to claim 1, **characterised in that** purified vWF is separated into high-molecular vWF and low-molecular vWF.

5. Process for separating vWF into high-molecular vWF and low-molecular vWF according to one of claims 1 to 4, **characterised in that** the separation is carried out in a Ca²⁺-free buffer system.

6. Process for separating vWF according to one of claims 1 to 5, **characterised in that** the affinity support is a support with heparin bound thereto, wherein AF-heparin Toyopearl® (Tosohaas), Heparin EVE)-Fraktogel® (Merck) and Heparin Sepharose Fast Flow® (Pharmacia), are equally preferably suitable.

7. Process for separating vWF according to one of claims 1 to 6, **characterised in that** a buffer solution consisting of buffer substances and optionally salt is used as a buffer system for affinity chromatography.

8. Process for separating vWF according to claim 7, **characterised in that** a buffer solution consisting of tris/HCl buffer, phosphate buffer or citrate buffer, and optionally salt, is used as a buffer system.

9. Process for separating vWF according to one of claims 1 to 8, **characterised in that** affinity chromatography is carried out in a pH range from 6.0 to 8.5, preferably at a pH value of 7.4.

10. Process according to one of claims 1 to 9, **characterised in that** low-molecular vWF is eluted at a lower salt concentration than high-molecular vWF.

11. Process according to one of claims 1 to 10, **characterised in that** the vWF is bound to the affinity support at a salt concentration < 150 mM, low-molecular aggregates of vWF are eluted at a salt concentration between 150 and 250 mM, preferably at 160 mM, and then high-molecular aggregates of vWF are eluted at a salt concentration of > 250 mM, preferably ≥ 270 mM.

12. Process according to one of claims 1 to 11, **characterised in that** soluble monovalent and divalent salts are used as salts.

13. Process according to claim 12, **characterised in that** NaCl is used as salt.

14. Composition for medicinal use, **characterised in that** it contains a vWF preparation of purified low-molecular vWF molecules with at least 83% of vWF dimers and a maximum 16% of vWF tetramers and a maximum 1% of higher polymers, and a physiologically acceptable support.

15. Composition for medicinal use according to claim 14, **characterised in that** it contains recombinant vWF, which consists of identical vWF sub-units.

16. Composition for medicinal use according to one of claims 14 or 15, **characterised in that** the vWF preparation is free of platelets having aggregating effect and binds factor VIII, contributes to the stabilisation of factor VIII, and positively influences the storage stability of factor VIII.

17. Composition for medicinal use, **characterised in that** the composition:
- contains a recombinant vWF preparation consisting of identical vWF sub-units, wherein the recombinant vWF preparation contains purified high-molecular vWF molecules, wherein high-molecular vWF molecules are those which have an activity which is improved by at least 50%, preferably at least 60%, compared to the physiological mixture of high-molecular and low-molecular vWF molecules per µg of protein in platelet aggregation, and
- a physiologically acceptable support.

18. Composition for medicinal use according to one of claims 14 to 17, **characterised in that** it contains a factor VIII or functional deletion mutant(s) of factor VIII.

19. Use of a medicinal composition according to one of claims 14 to 18, for producing a medicament for treating haemophilia A or various forms of von Willebrand's disease.

## Revendications

1. Procédé de séparation du facteur von Willebrand vWF, en fractions vWF à hauts poids moléculaires et vWF à bas poids moléculaires, **caractérisé en ce que** l'on lie le vWF à un support d'affinité, puis l'on élue à des concentrations en sel différentes.

2. Procédé de séparation de vWF selon la revendication 1, **caractérisé en ce que** le vWF à séparer se présente sous la forme d'une fraction de plasma, enrichie en vWF.

3. Procédé de séparation selon la revendication 1, **caractérisé en ce que** le vWF à séparer est un vWF recombinant, se présentant dans un concentrat de vWF recombinant, formé de surnageants de culture, exempts de cellules, de cellules transformées.

4. Procédé de séparation selon la revendication 1, **caractérisé en ce que** l'on sépare le vWF épuré, en vWF à hauts poids moléculaires et à bas poids moléculaires.

5. Procédé de séparation de vWF en vWF à hauts poids moléculaires et en vWF à bas poids moléculaires, selon l'une des revendications 1 à 4, **caractérisé en ce que** l'on effectue la séparation dans un système tampon, exempt de Ca²⁺.

6. Procédé de séparation de vWF selon l'une des revendications 1 à 5, **caractérisé en ce que** le support d'affinité est un support auquel est lié de l'héparine, de préférence, convenant à un degré identique, la AF-héparine Toyopearl^{®} (Tosohaas), l'héparine EMD-Fraktogel^{®} (Merck) et l'héparine Sepharose Fast Flow^{®} (Pharmacia).

7. Procédé de séparation de vWF selon l'une des revendications 1 à 6, **caractérisé en ce que** l'on utilise comme système tampon pour la chromatographie par affinité une solution tampon, formée de substances tampons et, le cas échéant, d'un sel.

8. Procédé de séparation de vWF selon la revendication 7, **caractérisé en ce qu'**on utilise comme système tampon une solution tampon, formée d'un tampon tris/HCL, d'un tampon au phosphate ou d'un tampon au citrate et, le cas échéant, de sel gris.

9. Procédé de séparation de vWF selon l'une des revendications 1 à 8, **caractérisé en ce que** la chromatographie par affinité est effectuée dans une plage de pH, allant de 6,0 à 8,5, de préférence à une valeur de pH de 7,4.

10. Procédé selon l'une des revendications 1 à 9, **caractérisé en ce qu'**on élue des vWF à bas poids moléculaires, pour une faible concentration en sel, en tant que vWF à hauts poids moléculaires.

11. Procédé selon l'une des revendications 1 à 10, **caractérisé en ce que,** pour une concentration en sel < 150 mM, on lie le vWF au support d'affinité, pour une concentration en sel comprise entre 150 et 250 mM, de préférence de 160 mM, on élue les agrégats à bas poids moléculaires du vWF et, ensuite, pour une concentration en sel > 250 mM, de préférence, ≥ 270 mM, on élue des agrégats à hauts poids moléculaires du vWF.

12. Procédé selon l'une des revendications 1 à il, **caractérisé en ce que** l'on utilise comme sels des sels mono- et bivalents solubles.

13. Procédé selon la revendication 12, **caractérisé en ce qu'**on utilise du NaCl en tant que sel.

14. Composition pour utilisation médicale, **caractérisée en ce qu'**elle contient une préparation de vWF, formée de molécules de vWF à bas poids moléculaires épurés, comprenant au moins 83 % de dimères de vWF et au plus de 16 % de tétramère de vWF et au plus de 1 % de polymères supérieurs, et un support physiologiquement acceptable.

15. Composition pour utilisation médicale selon la revendication 14, **caractérisée en ce qu'**elle contient un vWF recombinant, formé d'unités secondaires de vWF identiques.

16. Composition pour utilisation médicale selon l'une des revendications 14 ou 15, **caractérisée en ce que** la préparation de vWF est exempte d'un effet agrégeant les plaquettes et lie le facteur VIII, contribue à la stabilisation du facteur VIII et influe de façon positive sur la stabilité au stockage du facteur VIII.

17. Composition pour utilisation médicale, **caractérisée en ce que** la composition :
- contient une préparation recombinante de vWF, composée de sous-unités de vWF identiques, la préparation recombinante de vWF contenant des molécules de vWF à hauts poids moléculaires épurés, les molécules vWF à hauts poids moléculaires étant des molécules qui présentent une activité améliorée d'au moins 50 %, de préférence d'au moins 60 %, en comparaison avec le mélange physiologique de molécules vWF à hauts poids moléculaires et bas poids moléculaires par µg de protéine dans l'agrégation des plaquettes, et
- un support physiologiquement acceptable.

18. Composition pour utilisation médicale selon l'une des revendications 14 à 17, **caractérisée en ce qu'**elle contient un facteur VIII ou un ou des mutant(s) de délétion fonctionnelle du facteur VIII.

19. Utilisation d'une composition médicale selon l'une des revendications 14 à 18, pour la préparation d'un médicament pour le traitement de l'hémophilie A ou de différentes formes de la maladie de Willebrand
